Europäisches Patentamt

European Patent Office

Office européen des brevets

(11) Veröffentlichungsnummer: **0 047 958**

**A1**

(12)                    **EUROPÄISCHE PATENTANMELDUNG**

(21) Anmeldenummer: 81107039.0

(22) Anmeldetag: 07.09.81

(51) Int. Cl.³: **A 61 G 7/10**
**//A61G13/00, A61B6/04**

(30) Priorität: 16.09.80 DE 3034932

(43) Veröffentlichungstag der Anmeldung:
24.03.82 Patentblatt 82/12

(84) Benannte Vertragsstaaten:
DE FR GB IT

(71) Anmelder: SIEMENS AKTIENGESELLSCHAFT Berlin
und München
Postfach 22 02 61
D-8000 München 22(DE)

(72) Erfinder: Bär, Ulrich
Hermann-Köhl-Weg 15
D-8500 Nürnberg(DE)

(72) Erfinder: Uhl, Rudolf
Albrecht-Dürer-Strasse 14
D-8520 Erlangen(DE)

(54) Patientenlagerungsvorrichtung.

(57) Die Erfindung bezieht sich auf eine Patientenlagerungsvorrichtung mit einer von einem Fahrgestell (4) auf ein
Tischgestell (1, 2) umsetzbaren Lagerungsplatte (3) und mit
durch das Fahrgestell (4) betätigbaren Kupplungsmitteln (13,
14) für die Ankupplung der Lagerungsplatte (3) an das
Tischgestell (1, 2). Das Tischgestell (1, 2) weist einen sich auf
dem Fußboden abstützenden Sockel (1) und das Fahrgestell
(4) einen U-förmigen Rahmen (10, 11, 12) auf, der beim
Heranfahren des Fahrgestelles (4) an den Sockel (1) diesen
außen umgreift. Der Rahmen (10, 11, 12) und der Sockel (1)
sind mit zueinander passenden Führungsmitteln (15, 16) für
die Zentrierung und Verriegelung des Fahrgestelles (4) in
bezug auf den Sockel (1) versehen.

EP 0 047 958 A1

SIEMENS AKTIENGESELLSCHAFT     Unser Zeichen
Berlin und München               VPA 80 P 5120   E

-7-

Patientenlagerungsvorrichtung

Die Erfindung betrifft eine Patientenlagerungsvorrichtung mit einer von einem Fahrgestell auf ein Tischgestell umsetzbaren Lagerungsplatte und mit durch das Fahrgestell betätigbaren Kupplungsmitteln für die Ankupplung der Lagerungsplatte an das Tischgestell.

Eine Patientenlagerungsvorrichtung dieser Art ist in der DE-PS 28 12 074 beschrieben. Diese Patientenlagerungsvorrichtung ermöglicht es, einen Patienten auf einer auf dem Fahrgestell ruhenden Tischplatte für eine Untersuchung vorzubereiten und erst dann auf das Tischgestell aufzulegen, wenn die eigentliche Untersuchung, beispielsweise eine Röntgenuntersuchung, erfolgen soll. In Verbindung mit einem Tischgestell und mehreren Lagerungsplatten ist daher eine rationelle Ausnutzung der dem Tischgestell zugeordneten Untersuchungseinrichtung möglich. Die Vorbereitung für eine Untersuchung blockiert die Untersuchungseinrichtung nicht.

Die bekannte Patientenlagerungsvorrichtung weist ein Tischgestell auf, das mit einer sich auf dem Fußboden abstützenden Säule versehen ist, an der die Lagerungsplatte seitlich angeordnet wird. Ein derartiges Tischgestell ist jedoch nur für bestimmte Untersuchungen, insbesondere urologische Untersuchungen, geeignet. Für eine Reihe von Untersuchungsarten ist es erforderlich, ein Tischgestell vorzusehen, das einen sich auf dem Fußboden abstützenden Sockel aufweist, auf dessen Ober-

Tp 5 Ler / 28.08.1980

seite die Lagerungsplatte angeordnet wird, so daß eine
auf dem Tischgestell gelagerte Lagerungsplatte beispielsweise in die Öffnung eines Computertomographen
eingefahren werden kann. Für ein Tischgestell dieser
Art eignet sich das Fahrgestell bei der bekannten Patientenlagerungsvorrichtung nicht.

Der Erfindung liegt die Aufgabe zugrunde, eine Patientenlagerungsvorrichtung der eingangs genannten Art zu
schaffen, deren Anwendungsbereich nicht auf ein an einer Säule gelagertes Tischgestell beschränkt ist und
die sich insbesondere für die Anwendung bei einem Computertomographen eignet.

Diese Aufgabe ist erfindungsgemäß dadurch gelöst, daß
das Tischgestell einen sich auf dem Fußboden abstützenden Sockel und das Fahrgestell einen U-förmigen Rahmen
aufweist, der beim Heranfahren des Fahrgestelles an den
Sockel diesen außen umgreift und daß der Rahmen und der
Sockel mit zueinander passenden Führungsmitteln für die
Zentrierung des Fahrgestelles in bezug auf den Sockel
versehen sind. Bei der erfindungsgemäßen Patientenlagerungsvorrichtung kann eine Lagerungsplatte auf der
Oberseite eines sich auf dem Fußboden abstützenden
Sockels abgelegt werden. Der Sockel kann dabei gegebenenfalls höhenverstellbar sein. Auf diese Weise ist es
in Verbindung mit einem Computertomographen möglich,
einen Patienten auf dem Fahrgestell in einem Vorbereitungsraum für einen computertomographische Untersuchung vorzubereiten und erst für die eigentliche Untersuchung auf dem Sockel der Patientenliege des Computertomographen zu lagern.

Einzelheiten und zweckmäßige Weiterbildungen der Erfindung ergeben sich aus den Unteransprüchen.

- 3 -        VPA 80 P 5120   E

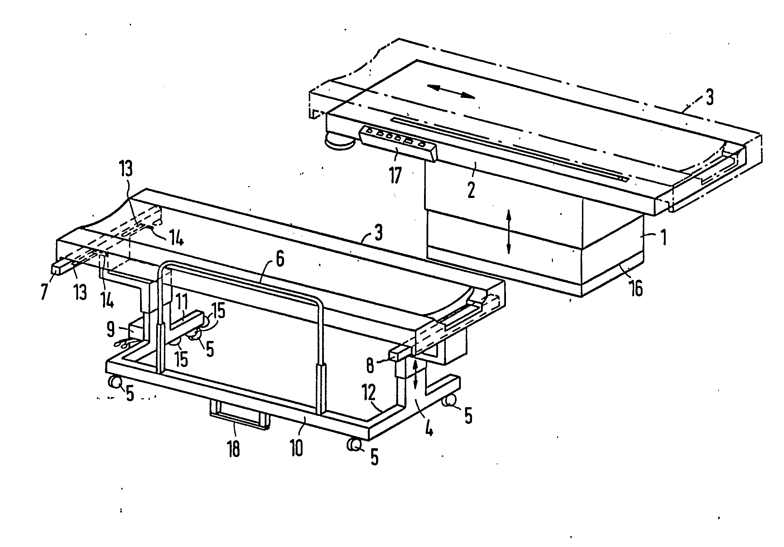

Die Erfindung ist nachfolgend anhand eines in der Zeichnung dargestellten Ausführungsbeispieles näher erläutert.

In der Zeichnung ist ein Sockel 1 eines Tischgestelles gezeichnet, auf dem ein Oberteil 2 höhenverstellbar angeordnet ist. Mit dem Oberteil 2 kann eine Lagerungsplatte 3 gemäß der strichpunktierten Darstellung fest verbunden werden. Der Sockel 1 bildet mit dem Oberteil 2 das Tischgestell eines Computertomographen, das es erlaubt, die Lagerungsplatte 3 in Längsrichtung zu verschieben und damit den Patienten in die Untersuchungsöffnung des Computertomographen so weit hineinzufahren, daß die gewünschte Transversalschicht abgetastet wird.

Für die Vorbereitung eines Patienten für eine computertomographische Untersuchung ist ein Fahrgestell 4 vorhanden, das mittels Räder 5 auf dem Fußboden verfahrbar ist. Das Fahrgestell 4 weist einen Handgriff 6 für das Verfahren des Patienten sowie auf seiner Oberseite Auflager 7, 8 für eine Tischplatte 3 auf. Die Tischplatte 3 ist auf dem Fahrgestell 4 mit Hilfe eines Hubmechanismus 9 höhenverstellbar.

Das Fahrgestell 4 besitzt - von oben gesehen - einen U-förmigen Rahmen mit einem Mittelteil 10 und zwei Schenkeln 11 und 12, die die Auflager 7, 8 tragen.

Jedes der Auflager 7, 8 ist seitlich mit Schrägflächen 13 versehen, die mit nach außen ragenden Zapfen 14 an der Unterseite der Lagerungsplatte 3 zusammenarbeiten und diese mit dem Oberteil 2 verriegeln.

Für die Vorbereitung eines Patienten für eine Untersuchung wird auf dem Fahrgestell 4, von dem in Verbin-

0047958

- 4 -        VPA 80 P 5120  E

dung mit einem Tischgestell mehrere vorhanden sein können, eine Lagerungsplatte 3, von der ebenfalls für ein Tischgestell mehrere vorgesehen sein können, auf gesetzt. Danach wird der Patient auf der Lagerungsplatte 3 vorbereitet und nach Abschluß der Vorbereitung wird das Fahrgestell 4 mit Hilfe des Handgriffes 6, der parallel zum Mittelteil 10 zwischen den beiden Schenkeln 11 und 12 verläuft, an den Sockel 1 herangefahren und so gegen den Sockel 1 geschoben, daß es mit Hilfe von seitlich an den Schenkeln 11, 12 nach innen gerichteten Rollen 15 und diesen zugeordneten Schienen 16 am Sockel 1 in bezug auf den Sockel 1 zentriert wird. Anschlagmittel stoppen die Bewegung des Fahrgestelles 4, wenn die Tischplatte 3 genau über dem Oberteil 2 liegt. Das Fahrgestell 4 wird beim Anfahren an die Anschlagmittel mit dem Sockel 1 verriegelt. Voraussetzung für das Heranfahren des Fahrgestelles 4 mit einer Lagerungsplatte 3 ist, daß das Oberteil 2 seine unterste Stellung einnimmt, in der es bei an den Sockel 1 herangefahrenen Fahrgestell 4 unterhalb der Lagerungsplatte 3 liegt.

Wird nunmehr durch Bedienungselemente an einem Bedienkästchen 17 das Oberteil 2 hochgefahren, so nimmt es die Lagerungsplatte 3 mit dem Patienten mit. Anschliessend kann das Fahrgestell 4 von Hand entfernt und für die Vorbereitung eines weiteren Patienten in Verbindung mit einer weiteren Lagerungsplatte 3 benutzt werden.

Ist eine Untersuchung beendet und soll die Lagerungsplatte 3 mit dem Patienten vom Oberteil 2 des Sockels 1 entfernt werden, so wird wieder das Fahrgestell 4 an den Sockel 1 herangefahren, so daß es diesen außen umgreift und genau unterhalb der Lagerungsplatte 3 liegt. Das Fahrgestell 4 wird dabei durch Andrücken an die Anschlagmittel mit dem Sockel 1 verriegelt. Anschließend wird die oberhalb der Auflager 7, 8 liegende Lagerungsplatte 3 abgesenkt. Wenn die nach außen ragenden Zapfen 14 die Schrägflächen 13 berühren, wird die Lagerungsplatte 3 vom Oberteil 2 abgekuppelt und auf die Auflager 7, 8 aufgelegt. Nach weiterem Absenken des Oberteiles 2 kann sie mit Hilfe des Fahrgestelles 4 vom Sockel 1 entfernt werden. Das Fahrgestell wird hierzu durch Betätigen eines Fußbügels 18 entriegelt.

1 Figur
4 Patentansprüche

Patentansprüche

1. Patientenlagerungsvorrichtung mit einer von einem Fahrgestell (4) auf ein Tischgestell (1, 2) umsetzbaren Lagerungsplatte (3) und mit durch das Fahrgestell (4) betätigbaren Kupplungsmitteln (14) für die Ankupplung der Lagerungsplatte (3) an das Tischgestell (1, 2), d a d u r c h   g e k e n n z e i c h n e t , daß das Tischgestell (1, 2) einen sich auf dem Fußboden abstützenden Sockel (1) und das Fahrgestell (4) einen U-förmigen Rahmen (10, 11, 12) aufweist, der beim Heranfahren des Fahrgestelles (4) an den Sockel (1) diesen außen umgreift und daß der Rahmen (10, 11, 12) und der Sockel (1) mit zueinander passenden Führungsmitteln (15, 16) für die Zentrierung des Fahrgestelles (4) in bezug auf den Sockel (1) versehen sind.

2. Patientenlagerungsvorrichtung nach Anspruch 1, d a d u r c h   g e k e n n z e i c h n e t ,   daß die Führungsmittel (15, 16) von nach innen gerichteten Rollen (15) an den beiden Schenkeln (11, 12) des Rahmens (10, 11, 12) und diesen zugeordneten Schienen (16) am Sockel (1) gebildet sind.

3. Patientenlagerungsvorrichtung nach Anspruch 1 oder 2,   d a d u r c h   g e k e n n z e i c h n e t , daß die Kupplungsmittel an der Tischplatte (3) verstellbar gelagerte, nach außen ragende Zapfen (14) aufweisen, die durch darauf einwirkende Schrägflächen (13) am Fahrgestell (4) betätigbar sind.

4. Patientenlagerungsvorrichtung nach einem der Ansprüche 1 bis 3,   d a d u r c h   g e k e n n - z e i c h n e t ,   daß das Fahrgestell (4) einen parallel zum Mittelteil (10) des Rahmens (10, 11, 12) verlaufenden Handgriff (6) aufweist.

0047958

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>US - A - 4 101 120</u> (SESHIMA)<br>* Spalte 5, Zeilen 25-44; Spalte 6, Zeilen 1-8; Figuren 1,5-7 *<br>-- | 1 |
| | <u>US - A - 4 105 923</u> (HYNES)<br>* Spalte 9, Zeilen 10-16; Figur 4 *<br>-- | 2 |
| | NEUES AUS DER TECHNICK, Nr. 6, 15. Dezember 1973,<br>* Ganze Röntgenunternehmungsvorrichtung und entsprechende Figur *<br>-- | 3 |
| A | <u>US - A - 2 681 839</u> (LIMBACH) | |
| A | <u>US - A - 3 504 386</u> (ROSSI) | |
| A | <u>GB - A - 961 677</u> (BANKS) | |
| A | <u>DE - A - 1 566 154</u> (SIEMENS)<br>---- | |

**KLASSIFIKATION DER ANMELDUNG (Int Cl ³)**

A 61 G   7/10//
A 61 G  13/00
A 61 B   6/04

**RECHERCHIERTE SACHGEBIETE (Int. Cl.³)**

A 61 G
A 61 B

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 03-12-1981 | MAROSCIA |

EPA form 1503.1   06.78